# FASCICULE DE BREVET EUROPEEN

(11) **EP 1 858 321 B1**
(45) Date de publication et mention de la délivrance du brevet: **10.10.2012**
(21) Numéro de dépôt: 06726078.6
(22) Date de dépôt: 13.03.2006
(51) Int. Cl.: A01N 25/34, A61L 2/23, A01N 25/08, A01N 25/12

(54) **PASTILLE DESINFECTANTE, COMPACTE ET SECABLE**
KOMPAKTE DESINFEKTIONSTABLETTE MIT BRUCHRILLE
SCORED, COMPACT DISINFECTANT TABLET

(30) Priorité: 15.03.2005 FR 0502546
(43) Date de publication de la demande: 28.11.2007
(73) Titulaire: Eurotab, 42170 Saint Just Saint Rambert (FR)
(72) Inventeur: RUBINSTENN, Gilles, F-75005 Paris (FR); DRIOT, Christine, F-42580 l'Etrat (FR)
(74) Mandataire: Brédeville, Odile Marie
(86) Numéro de dépôt international: PCT/FR2006/000549
(87) Numéro de publication internationale: WO 2006/097612

(56) Documents cités:
- WO-A-01/64035
- US-A- 4 288 430
- US-A- 5 180 587
- US-A1- 2003 118 472

## Description

La présente invention porte sur une pastille désinfectante compacte, à base d'un agent désinfectant, comme par exemple le chlore, sécable précisément en deux moitiés.

Dans le domaine de la désinfection des surfaces et des objets en contact avec les substances alimentaires, il est connu d'utiliser des produits compacts, par exemple sous forme de pastilles, à base d'un agent désinfectant, comme par exemple le chlore actif. Une telle pastille est appréciée pour sa facilité d'utilisation : en effet, il suffit de la diluer dans un certain volume d'eau pour obtenir un produit désinfectant prêt à l'emploi. Par ailleurs, du fait de sa forme compacte, une telle pastille est facile à manipuler et à stocker. Enfin, un tel conditionnement évite également les risques de projection de produits dangereux.

Ces pastilles sont en général prévues pour une dilution dans un volume d'eau prédéterminé, ce volume étant généralement conséquent. Or il peut arriver que, selon la surface à désinfecter ou l'utilisation souhaitée de la solution désinfectante, un tel volume de solution désinfectante ne soit pas nécessaire. Dans un tel cas, la pastille n'étant pas divisible de façon précise, on est contraint, afin de respecter les normes de désinfection, de l'utiliser entièrement, générant ainsi un excès de rejet du chlore dans l'environnement. Or, le chlore est une substance toxique, pour les organismes aquatiques en particulier. De plus, une telle opération représente également un gaspillage de la matière première chlore en tant que telle.

Par ailleurs, il peut arriver que l'utilisateur ait besoin dans certains cas d'une quantité importante de solution désinfectante.
Le document GB 1 387 643 décrit des pastilles comprenant deux surfaces opposées convexes, l'une des surfaces étant munie d'une barre de sécabilité.

Ainsi, il subsiste le besoin d'une pastille désinfectante à usage universel, c'est-à-dire pouvant convenir aussi bien à des situations où une forte quantité d'agent désinfectant est nécessaire qu'à des situations où une moindre quantité d'un tel agent est nécessaire, cette pastille permettant d'ajuster la consommation d'agent désinfectant et donc son rejet dans la nature en respectant ainsi au mieux l'environnement et en préservant au mieux les ressources naturelles, cette pastille respectant par ailleurs les normes en vigueur de désinfection.

La présente invention vise à remédier à ce problème en proposant une pastille désinfectante à base d'un agent désinfectant comme par exemple le chlore, qui présente à la fois une taille réduite et une excellente sécabilité, cette pastille permettant ainsi d'ajuster de façon précise la dose d'agent désinfectant, par exemple de chlore, au volume de solution désinfectante final souhaité tout en respectant les normes de désinfection.

La Demanderesse a en effet trouvé de façon surprenante qu'en appliquant une force de compression spécifique lors de la fabrication d'une pastille contenant un agent désinfectant, il était possible de réaliser une pastille de taille particulièrement réduite qui par ailleurs soit sécable très précisément, et ce selon une barre de sécabilité ménagée sur au moins une des surfaces de ladite pastille, cette barre de sécabilité indiquant une ligne de division en deux parties exactes, c'est-à-dire en deux moitiés, de la pastille. Une telle propriété permet d'extrapoler très précisément la dose d'agent désinfectant présente dans chaque moitié obtenue et permet ainsi d'utiliser chacune de ces moitiés avec une efficacité optimale, c'est-à-dire en respectant les normes de désinfection en vigueur. De plus, une telle pastille ne s'ébrèche pas lors de la fabrication à haute cadence, du conditionnement ou du transport, et est par ailleurs cassable aisément par l'utilisateur.

La présente invention porte sur une composition compacte sous forme de pastille, comprenant au moins un agent désinfectant, ladite pastille présentant une masse allant de 1,5 à 3 g et une hauteur allant de 2,5 à 7 mm, caractérisée en ce que :
- ladite pastille présente une résistance à la rupture allant de 55 à 95 N mesurée selon la méthode de la monographie 2.9.8 de la Pharmacopée Européenne,
- ladite pastille présentant sur au moins l'une de ses faces au moins une barre de sécabilité indiquant une ligne de division de la pastille en deux moitiés.

La présente invention porte également sur un procédé de fabrication d'une composition compacte sous forme de pastille sécable en deux moitiés sensiblement identiques, comprenant au moins un agent désinfectant, caractérisé en ce qu'il comprend les étapes suivantes :
- a°) on prépare 1,5 à 3 g d'un mélange de poudres comprenant au moins un agent désinfectant,
- b°) on compacte dans une pastilleuse le mélange obtenu en a°) à une force de compression allant de 20 à 35 kN pour obtenir la pastille, en imprimant sur au moins une des faces de la pastille au moins une barre de sécabilité indiquant la ligne de division de la pastille en deux moitiés.

La présente invention porte encore sur une composition compacte sous forme de pastille, susceptible d'être obtenue selon le procédé décrit ci-dessus.

La pastille selon l'invention présente à la fois une taille réduite et une excellente sécabilité. En particulier, la pastille selon l'invention se casse sans s'ébrécher. Il est donc possible de choisir de n'utiliser que la moitié de la pastille sans perdre de masse et donc sans perdre de matière active ce qui entraînerait une perte d'efficacité et le non respect des normes de désinfection. De même, la pastille selon l'invention garde son intégrité lors de la fabrication à haute cadence, pendant le conditionnement, le stockage, le transport.

La pastille selon l'invention permet de n'utiliser que la quantité d'agent désinfectant, par exemple de chlore, nécessaire pour des volumes finaux de solutions désinfectantes modérés. Elle permet également d'obtenir des volumes finaux de solutions désinfectantes plus conséquents si nécessaires. Elle permet donc d'ajuster précisément la quantité de chlore nécessaire : ainsi sont évités à la fois le gaspillage de chlore et le rejet excessif du même chlore dans l'environnement.

Par ailleurs, chaque moitié de pastille obtenue après cassure contenant précisément la moitié de la dose totale de la pastille en agent désinfectant, il est possible d'utiliser chacune de ces moitiés avec la meilleure efficacité possible.

La présente invention va maintenant être décrite plus en détails à l'aide de la description qui va suivre et du dessin annexé dans lequel :

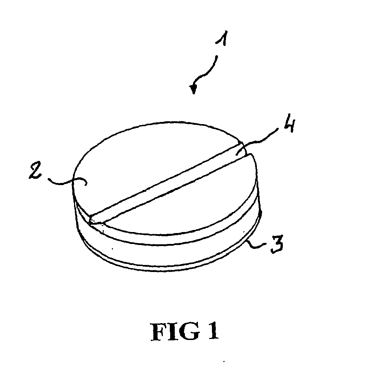

- la figure 1 est une vue en perspective d'une pastille selon l'invention.

Par « pastille », on entend, au sens de la présente demande, un produit compact en trois dimensions dont la largeur et la longueur sont du même ordre de grandeur, cet ordre de grandeur étant bien supérieur à celui de la hauteur. Ainsi, le terme « pastille » comprend les termes comprimé, tablette, galet, etc... Cette pastille peut présenter des faces supérieure et inférieure plates ou bombées, concaves ou convexes. Cette pastille peut être indifféremment de forme générale ronde, ovale, carrée, rectangulaire, octogonale, polygonale.

La pastille selon l'invention comprend au moins une barre de sécabilité ménagée sur au moins une de ses faces, par exemple la face inférieure ou la face supérieure, cette barre de sécabilité indiquant une ligne de division de la pastille en deux moitiés, c'est-à-dire en deux parties exactes.

Par exemple, dans le cas où la pastille selon l'invention est ronde, la barre de sécabilité est ménagée selon un diamètre de la pastille. Dans le cas où la pastille est carrée ou rectangulaire, la barre de sécabilité peut être ménagée selon une diagonale du carré ou du rectangle.

La barre de sécabilité peut se présenter sous toute forme indiquant une ligne de division de la pastille en deux moitiés. Par exemple, la barre de sécabilité peut être un simple trait tracé sur une des faces de la pastille. La barre de sécabilité peut encore être sous la forme d'une légère rainure ou entaille ménagée selon une ligne de division de la pastille en deux moitiés. Dans une forme préférée de réalisation de l'invention, la barre de sécabilité se présente sous la forme d'une rainure en creux de section triangulaire équilatérale d'environ 0,5 mm de côté.

A titre d'exemple, sur la figure 1 est représentée une pastille 1 selon l'invention, de forme générale ronde. Cette pastille 1 comprend une face supérieure 2 et une face inférieure 3. Sur l'exemple représenté, la pastille 1 comprend une barre de sécabilité 4 sur sa face supérieure 2, cette barre de sécabilité étant ici ménagée selon un diamètre de la pastille et indiquant ainsi une ligne de division de la pastille 1 en deux moitiés. Sur l'exemple représenté, la barre de sécabilité 4 se présente sous la forme d'une rainure en creux de section triangulaire équilatérale d'environ 0,5 mm de côté.

La pastille selon l'invention peut également comprendre des barres de sécabilité additionnelles, comme par exemple des barres indiquant une ligne de division en deux moitiés de chacune des moitiés de la pastille.

La composition compacte selon l'invention comprend au moins un agent désinfectant. De préférence, l'agent désinfectant est choisi parmi les composés libérant du chlore actif au contact de l'eau.

L'agent désinfectant peut être choisi parmi le dichloroisocyanurate de sodium dihydraté, le sel de sodium de N-chloro-4-méthylbenzène sulfonamide sous forme anhydre ou dihydratée, le sel de sodium de 1,3-dichloro-s-triazine-2,4,6 trione sous forme anhydre ou dihydratée, et leurs mélanges. De préférence, l'agent désinfectant est le dichloroisocyanurate de sodium dihydraté un tel produit est disponible commercialement sous le nom commercial « ACl 56 » auprès de la sociéte Oxychem.

De préférence l'agent désinfectant est présent dans la composition à une teneur allant de 50 à 99,9% en poids, de préférence de 60 à 99,9% en poids, par rapport au poids total de la composition. De préférence cette teneur va de 70 à 99,9% en poids, de préférence de 75 à 85% en poids, par rapport au poids total de la composition.

Ainsi, de préférence, la composition selon l'invention comprend de 25 à 60 % en poids, par rapport au poids total de la composition, de chlore actif.

La composition selon l'invention peut comprendre en outre au moins un système effervescent.

De préférence le système effervescent est présent dans la composition à une teneur allant de 1 à 30%, en poids, de préférence de 2 à 25% en poids, de préférence encore de 5 à 20% en poids, et de préférence encore de 10 à 20% en poids, par rapport au poids total de la composition.

Le système effervescent peut comprendre l'association d'au moins un acide et d'au moins une base carbonatée.

De préférence le système effervescent comprend l'association d'acide adipique et d'hydrogénocarbonate de sodium.

La composition selon l'invention peut comprendre en outre au moins un agent désintégrant.

De préférence l'agent désintégrant comprend au moins un polymère réticulé choisi parmi les dérivés de polyacrylates, les dérivés de cellulose et leurs mélanges.

De préférence l'agent désintégrant est la carboxyméthyl cellulose réticulée.

De préférence, l'agent désintégrant est présent dans la composition à une teneur pouvant aller jusqu'à 10% en poids, par rapport au poids total de la composition.

Ces agents désinfectants, effervescents et désintégrants sont de préférence fournis sous forme de poudre.

La composition selon l'invention peut comprendre en outre des composés additionnels comme des agents liants, des tensioactifs anioniques, des tensioactifs cationiques, des tensioactifs non ioniques, des systèmes complexants, des dispersants, des enzymes, des colorants, des parfums, et leurs mélanges.

La pastille selon l'invention est de préférence préparée selon le procédé comprenant les étapes suivantes :
a°) on prépare 1,5 à 3 g d'un mélange de poudres comprenant au moins un agent désinfectant,
   - b°) on compacte dans une pastilleuse le mélange obtenu en a°) à une force de compression allant de 20 à 35 kN pour obtenir la pastille, en imprimant sur au moins une des faces de la pastille au moins une barre de sécabilité indiquant une ligne de division de la pastille en deux moitiés.

Les pastilles selon l'invention présentent une résistance à la rupture allant de 55 à 95 N et de préférence encore allant de 65 à 85 N.

Dans la présente demande, la résistance à la rupture est mesurée selon la méthode suivante : on mesure, à l'aide d'un duromètre, par exemple le duromètre « Schleuniger 8M » la force nécessaire à appliquer pour briser la pastille, encore appelée résistance à la rupture (en Newton), selon le test défini dans la Pharmacopée Européenne dans la monographie 2.9.8.

Dans la présente demande, on définit un taux de facilité à casser la pastille en deux. Ce taux est évalué de la manière suivante : dix pastilles de compositions identiques sont fabriquées selon le procédé décrit ci-dessus, avec la même force de compression. On imprime à chacune de ces pastilles une barre de sécabilité identique. Ces pastilles sont ensuite cassées en deux selon cette barre de sécabilité. Pour chacune d'elles, les deux morceaux obtenus sont pesés. Le pourcentage de différence par rapport à la moitié exacte théorique est calculé et la moyenne est faite sur les trois plus mauvaises valeurs. Cette moyenne, ou taux de facilité à casser la pastille en deux, indique la qualité de la cassure.

Si le taux de facilité à casser la pastille en deux est compris entre 0 et 0,25, bornes incluses, la cassure est nette et les deux moitiés obtenues sont pratiquement ou sensiblement identiques. Il est alors possible d'effectuer un dosage très précis de solution désinfectante à l'aide de l'une ou de l'autre moitié obtenue après cassure.

Si le taux de facilité à casser la pastille en deux est strictement supérieur à 0,25, alors la cassure n'est pas nette, et les deux parties obtenues présentent une masse sensiblement différente. Il n'est alors pas possible d'extrapoler la dose d'agent désinfectant contenue dans chaque partie obtenue après cassure.

La présente invention va maintenant être illustrée à l'aide des exemples suivants.

### EXEMPLE 1 : (selon l'invention)

Une composition compacte selon l'invention de formulation suivante a été réalisée (les quantités sont données en pourcentage de poids par rapport au poids total de la composition) :
- acide adipique fourni par la société BASF 7,69%
- hydrogénocarbonate de sodium fourni par la société SOLVAY 11, 54%
- dichloroisocyanurate de sodium vendu sous la dénomination commerciale « ACl 56 » par la société Oxychem 80,77%
   TOTAL 100%

Ces matières premières, sous forme de poudre, sont mélangées. 250 kg de ce mélange est compacté dans une pastilleuse de type presse rotative (« Fette Perfect 3 ») équipée de poinçons de diamètre 20 mm, dont les poinçons supérieurs présentent une barre de sécabilité standard à section triangulaire équilatérale de 0,5 mm. La force de compression utilisée est de 25 kN. Environ 13500 pastilles sont obtenues.

On obtient des pastilles rondes, chaque pastille présentant les caractéristiques suivantes :
- masse : 1,8 g
- hauteur 3,7 mm
- diamètre 20 mm
- résistance à la rupture 70 N

La barre de sécabilité de cette pastille est ménagée selon un diamètre de la face supérieure de la pastille. Elle se présente sous la forme d'une rainure en creux de section triangulaire équilatérale d'environ 0,5 mm de côté.

La pastille réalisée selon cet exemple se casse très nettement et très facilement selon son diamètre au niveau de la barre de sécabilité.

Cette pastille présente un taux de facilité à casser la pastille en deux, mesuré comme indiqué ci-dessus, d'environ 0,16.

Ainsi, la pastille selon l'invention se casse aisément selon sa barre de sécabilité et on peut très facilement et rapidement être en présence de deux doses d'agent désinfectant dont il est possible d'extrapoler la valeur sans faire d'erreur grossière.

Ainsi, il a été vérifié que :
- utilisée dans son intégralité dans un volume de 10 litres d'eau propre, la pastille de l'exemple 1 permet d'obtenir une activité bactéricide selon la norme EN 1276 ;
- chaque moitié, obtenue après cassure de la pastille en deux, utilisée chacune dans un volume de 5 litres d'eau propre, permet d'obtenir une activité bactéricide selon la norme EN 1276.

Ainsi, la pastille selon cet exemple permet d'obtenir un dosage précis aussi bien pour 10 litres d'eau que pour 5 litres d'eau.

La pastille selon l'invention, du fait qu'elle est parfaitement sécable en deux, permet de s'adapter au volume de solution désinfectante final souhaité et ainsi de préserver les ressources naturelles ainsi que l'environnement en évitant le rejet excessif de chlore dans la nature.

De plus, la pastille selon cet exemple ne s'ébrèche pas lors de la fabrication à haute cadence ou pendant le transport et le stockage.

### EXEMPLE 2 (comparatif) :

Une pastille de formulation identique à celle de la pastille de l'exemple 1 a été fabriquée. Le procédé de fabrication appliqué était celui de l'exemple 1 mais la force de compression utilisée pour compacter la composition était de 15 kN.

On obtient une pastille ronde présentant les caractéristiques suivantes :
- masse : 1,8 g
- hauteur 3,8 mm
- diamètre 20 mm
- résistance à la rupture 50 N

Comme pour l'exemple 1, la barre de sécabilité de cette pastille est ménagée selon un diamètre de la face supérieure de la pastille. Elle se présente sous la forme d'une rainure en creux de section triangulaire équilatérale d'environ 0,5 mm de côté.

Le taux de facilité à casser la pastille en deux a été mesuré comme indiqué ci-dessus. Il est d'environ 0,3.

Ainsi, cette pastille ne se casse pas nettement selon sa barre de sécabilité ; elle s'ébrèche et il n'est pas possible d'extrapoler la dose d'agent désinfectant présent dans chaque partie de la pastille après cassure.

### EXEMPLE 3 : (comparatif)

Une pastille de formulation identique à celle de la pastille de l'exemple 1 a été fabriquée. Le procédé de fabrication appliqué était celui de l'exemple 1 mais la force de compression utilisée pour compacter la composition était de 40 kN.

On obtient une pastille ronde présentant les caractéristiques suivantes :
- masse : 1,8 g
- hauteur 3,6 mm
- diamètre 20 mm
- résistance à la rupture 125 N

Comme pour l'exemple 1, la barre de sécabilité de cette pastille est ménagée selon un diamètre de la face supérieure de la pastille. Elle se présente sous la forme d'une rainure en creux de section triangulaire équilatérale d'environ 0,5 mm de côté.

Le taux de facilité à casser la pastille en deux a été mesuré comme indiqué ci-dessus. Il est d'environ 0,27.

Ainsi, cette pastille est très difficile à casser. Une force importante est nécessaire et il est difficile de casser la pastille selon son diamètre, même en présence d'une barre de sécabilité.

## Revendications

1. Composition compacte sous forme de pastille, comprenant au moins un agent désinfectant, ladite pastille présentant une masse allant de 1,5 à 3 g et une hauteur allant de 2,5 à 7 mm,
**caractérisée en ce que** :
- ladite pastille présente une résistance à la rupture allant de 55 à 95 N mesurée selon la méthode de la monographie 2.9.8 de la Pharmacopée Européenne,
- ladite pastille présentant sur au moins l'une de ses faces au moins une barre de sécabilité indiquant une ligne de division de la pastille en deux moitiés.

2. Composition selon la revendication 1, **caractérisée en ce que** l'agent désinfectant est choisi parmi les composés libérant du chlore actif au contact de l'eau.

3. Composition selon la revendication 2, **caractérisée en ce que** l'agent désinfectant est choisi parmi le dichloroisocyanurate de sodium dihydraté, le sel de sodium de N-chloro-4-méthylbenzène sulfonamide sous forme anhydre ou dihydratée, le sel de sodium de 1,3-dichloro-s-triazine-2,4,6 trione sous forme anhydre ou dihydratée, et leurs mélanges.

4. Composition selon l'une quelconque des revendications 1 à 3, **caractérisée en ce que** l'agent désinfectant est présent dans la composition à une teneur allant de 50 à 99,9% en poids, de préférence de 60 à 99,9% en poids, par rapport au poids total de la composition.

5. Composition selon la revendication précédente, **caractérisée en ce que** cette teneur va de 70 à 99,9% en poids, de préférence de 75 à 85% en poids, par rapport au poids total de la composition.

6. Composition selon l'une quelconque des revendications précédentes, **caractérisée en ce qu'**elle comprend en outre au moins un système effervescent.

7. Composition selon la revendication précédente, **caractérisée en ce que** le système effervescent est présent dans la composition à une teneur allant de 1 à 30%, en poids, de préférence de 2 à 25% en poids, de préférence encore de 5 à 20% en poids, et de préférence encore de 10 à 20% en poids, par rapport au poids total de la composition.

8. Composition selon l'une quelconque des revendications 6 ou 7, **caractérisée en ce que** le système effervescent comprend l'association d'au moins un acide et d'au moins une base carbonatée.

9. Composition selon la revendication précédente, **caractérisée en ce que** le système effervescent comprend l'association d'acide adipique et d'hydrogénocarbonate de sodium.

10. Composition selon l'une quelconque des revendications précédentes, **caractérisée en ce qu'**elle comprend en outre au moins un agent désintégrant.

11. Composition selon la revendication précédente, **caractérisée en ce que** l'agent désintégrant comprend au moins un polymère réticulé choisi parmi les dérivés de polyacrylates, les dérivés de cellulose et leurs mélanges.

12. Composition selon la revendication précédente, **caractérisée en ce que** l'agent désintégrant est la carboxyméthyl cellulose réticulée.

13. Composition selon la revendication 10 ou 11, caractérisée en ce l'agent désintégrant est présent dans la composition à une teneur pouvant aller jusqu'à 10% en poids, par rapport au poids total de la composition.

14. Composition selon l'une quelconque des revendications précédentes, **caractérisée en ce que** ladite résistance à la rupture va de 65 à 85 N.

15. Composition selon l'une quelconque des revendications précédentes, **caractérisé en ce que** qu'elle comprend de 25 à 60 % en poids, par rapport au poids total de la composition, de chlore actif.

16. Composition selon l'une quelconque des revendications précédentes, **caractérisée en ce que** la barre de sécabilité se présente sous la forme d'une rainure en creux de section triangulaire équilatérale d'environ 0,5 mm de côté.

17. Procédé de fabrication d'une composition compacte sous forme de pastille sécable en deux moitiés sensiblement identiques, comprenant au moins un agent désinfectant, **caractérisé en ce qu'**il comprend les étapes suivantes :
- a°) on prépare 1,5 à 3 g d'un mélange de poudres comprenant au moins un agent désinfectant,
- b°) on compacte dans une pastilleuse le mélange obtenu en a°) à une force de compression allant de 20 à 35 kN pour obtenir la pastille, en imprimant sur au moins une des faces de la pastille au moins une barre de sécabilité indiquant une ligne de division de la pastille en deux moitiés.

## Claims

1. A compact composition as a tablet, comprising at least one disinfecting agent, said tablet having a mass ranging from 1.5 to 3 g, and a height ranging from 2.5 to 7 mm,
**characterized in that**:
- said tablet has a breaking strength ranging from 55 to 95 N, according to the method of monograph 2.9.8 of the European Pharmacopoeia,
- said tablet having on at least one of its faces at least one breakable bar indicating a line for dividing the tablet into two halves.

2. The composition according to claim 1, **characterized in that** the disinfecting agent is selected from compounds releasing active chlorine upon contact with water.

3. The composition according to claim 2, **characterized in that** the disinfecting agent is selected from sodium dichloroisocyanurate dihydrate, sodium N-chloro-4-methylbenzene sulfonamide salt in an anhydrous or dihydrate form, sodium 1,3-dichloro-s-triazine-2,4,6-trione salt in an anhydrous or dihydrate form, and mixtures thereof.

4. The composition according to any of claims 1 to 3, **characterized in that** the disinfecting agent is present in the composition at a content ranging from 50 to 99.9% by weight, preferably from 60 to 99.9% by weight, based on the total weight of the composition.

5. The composition according to the preceding claim, **characterized in that** this content ranges from 70 to 99.9% by weight, preferably from 75 to 85% by weight, based on the total weight of the composition.

6. The composition according to any of the preceding claims, **characterized in that** it further comprises at least one effervescent system.

7. The composition according to the preceding claim, **characterized in that** the effervescent system is present in the composition at a content ranging from 1 to 30% by weight, preferably from 2 to 25% by weight, still preferably from 5 to 20% by weight, and still preferably from 10 to 20% by weight, based on the total weight of the composition.

8. The composition according to any of claims 6 or 7, **characterized in that** the effervescent system comprises the combination of at least one acid and of at least one carbonated base.

9. The composition according to the preceding claim, **characterized in that** the effervescent system comprises the combination of adipic acid and of sodium hydrogen carbonate.

10. The composition according to any of the preceding claims, **characterized in that** it further comprises at least one disintegrating agent.

11. The composition according to the preceding claim, **characterized in that** the disintegrating agent comprises at least one cross-linked polymer selected from derivatives of polyacrylates, derivatives of cellulose, and mixtures thereof.

12. The composition according to the preceding claim, **characterized in that** the disintegrating agent is cross-linked carboxymethyl cellulose.

13. The composition according to claim 10 or 11, **characterized in that** the disintegrating agent is present in the composition at a content, which may range up to 10% by weight, based on the total weight of the composition.

14. The composition according to any of the preceding claims, **characterized in that** said breaking strength ranges from 65 to 85 N.

15. The composition according to any of the preceding claims, **characterized in that** it comprises from 25 to 60% by weight, based on the total weight of the composition, of active chlorine.

16. The composition according to the preceding claims, **characterized in that** the breakable bar appears as a recessed groove with an equilateral triangle section with a side of about 0.5 mm.

17. A method for making a compact composition as a tablet which may be split into two substantially identical halves, comprising at least one disinfecting agent, **characterized in that** it comprises the following steps:
- a°) 1.5 to 3 g of a mixture of powders, comprising at least one disinfecting agent is prepared,
- b°) the mixture obtained in a°) is contacted in a tabletting machine at a compression force ranging from 20 to 35 kN in order to obtain the tablet, by printing on at least one of the faces of the tablet at least one breakable bar indicating a line for dividing the tablet into two halves.

## Patentansprüche

1. Kompakte Zusammensetzung in Tablettenform, die mindestens ein Desinfektionsmittel umfasst, wobei die Tablette eine Masse von 1,5 bis 3 g und eine Höhe von 2,5 bis 7 mm aufweist,
**dadurch gekennzeichnet, dass**:
- die Tablette eine Bruchfestigkeitvon 55 bis 95 N aufweist, gemessen gemäß der Monographie-Methode 2.9.8 derEuropäischen Pharmakopöe,
- die Tablette auf mindestens einer ihrer Seiten eine Bruchlinieaufweist, die eine Teilungslinie der Tablette in zwei Hälften anzeigt.

2. Zusammensetzung nach Anspruch 1, **dadurch gekennzeichnet, dass** das Desinfektionsmittelaus den Verbindungen ausgewählt ist, die im Kontakt mit Wasser aktives Chlor freisetzen.

3. Zusammensetzung nach Anspruch 2, **dadurch gekennzeichnet, dass** das Desinfektionsmittelaus dem Natriumdichlorisocyanurat-Dihydrat, dem N-Chlor-4-methylbenzolsulfonamid Natriumsalz in wasserfreier oder Dihydrat-Form, dem 1,3-Dichlor-s-triazin-2,4,6 trionNatriumsalz inwasserfreier oder Dihydrat- Formund ihren Gemischen ausgewählt ist.

4. Zusammensetzung nach einem der Ansprüche 1 bis 3, **dadurch gekennzeichnet, dass** das Desinfektionsmittelin der Zusammensetzung in einem Gehalt von 50 bis 99,9Gew.-%, vorzugsweise von 60 bis 99,9Gew.-%, im Verhältnis zum Gesamtgewicht der Zusammensetzung vorhanden ist.

5. Zusammensetzung nach vorangehendem Anspruch, **dadurch gekennzeichnet, dass** dieser Gehalt von 70 bis 99,9Gew.-%, vorzugsweise von 75 bis 85Gew.-%,im Verhältnis zum Gesamtgewicht der Zusammensetzung reicht.

6. Zusammensetzung nach einem der vorangehenden Ansprüche, **dadurch gekennzeichnet, dass** sie ferner mindestens ein aufschäumendes System umfasst.

7. Zusammensetzung nach vorangehendem Anspruch, **dadurch gekennzeichnet, dass** das aufschäumende System in der Zusammensetzung in einem Gehalt von 1 bis 30Gew.-%, vorzugsweise von 2 bis 25Gew.-%, in noch bevorzugter Weise von 5 bis 20Gew.-% und in noch bevorzugter Weise von 10 bis 20Gew.-%,im Verhältnis zum Gesamtgewicht der Zusammensetzung vorhanden ist.

8. Zusammensetzung nach einem der Ansprüche 6 oder 7, **dadurch gekennzeichnet, dass** das aufschäumende System die Assoziation mindestens einer Säure und mindestens einer Carbonatbasis umfasst.

9. Zusammensetzung nach vorangehendem Anspruch, **dadurch gekennzeichnet, dass** das aufschäumende System die Assoziation vonAdipinsäure und von Natriumhydrogencarbonatumfasst.

10. Zusammensetzung nach einem der vorangehenden Ansprüche, **dadurch gekennzeichnet, dass** sie ferner mindestens ein Zerfallhilfsmittel umfasst.

11. Zusammensetzung nach vorangehendem Anspruch, **dadurch gekennzeichnet, dass** das Zerfallhilfsmittel mindestens ein vernetztes Polymer umfasst, das aus den Polyacrylat-Derivaten, den Cellulosederivaten und ihren Gemischen ausgewählt ist.

12. Zusammensetzung nach vorangehendem Anspruch, **dadurch gekennzeichnet, dass** das Zerfallhilfsmittel die vernetzte Carboxymethylcelluloseist.

13. Zusammensetzung nach Anspruch 10 oder 11, **dadurch gekennzeichnet, dass** dieses Zerfallhilfsmittel in der Zusammensetzung in einem Gehalt vorhanden ist, der bis zu 10Gew.-% im Verhältnis zum Gesamtgewicht der Zusammensetzung gehen kann.

14. Zusammensetzung nach einem der vorangehenden Ansprüche, **dadurch gekennzeichnet, dass** dieBruchfestigkeit von 65 bis 85 N reicht.

15. Zusammensetzung nach einem der vorangehenden Ansprüche, **dadurch gekennzeichnet, dass** sie 25 bis 60 Gew.-% aktives Chlor im Verhältnis zum Gesamtgewicht der Zusammensetzung umfasst.

16. Zusammensetzung nach einem der vorangehenden Ansprüche, **dadurch gekennzeichnet, dass** die Bruchliniedie Form einer hohlen Rille mit dem Querschnitt eines gleichseitigen Dreiecks von zirka 0,5 mm Seitenlänge hat.

17. Herstellungsverfahren einer kompakten Zusammensetzung in Form einer in zwei etwa gleiche Hälften zerbrechbarenTablette, die mindestens ein Desinfektionsmittel umfasst, **dadurch gekennzeichnet, dass** es die folgenden Schritte umfasst:
- a) Vorbereitung von 1,5 bis 3 g eines Pulvergemischs, das mindestens ein Desinfektionsmittel umfasst,
- b) Kompaktierung des bei Schritt a) gewonnenen Gemischs in einer Tablettenmaschine mit einer Kompressionskraft von 20 bis 35 kN, um die Tablette zu erhalten, bei Drucken von mindestens einer Bruchlinieauf mindestens einer der Seiten der Tablette, die die Teilungslinie der Tablette in zwei Hälften anzeigt.
